# EUROPEAN PATENT SPECIFICATION

(11) **EP 3 820 416 B1**
(45) Date of publication and mention of the grant of the patent: **06.09.2023**
(21) Application number: 19745518.1
(22) Date of filing: 10.07.2019
(51) Int. Cl.: A61F 5/441

(54) **OSTOMY POUCH FILTER**
STOMABEUTELFILTER
FILTRE POUR POCHE DE STOMIE

(30) Priority: 10.07.2018 US 201862696179 P
(43) Date of publication of application: 19.05.2021
(60) Divisional application: 23180556.5
(73) Proprietor: Hollister Incorporated, Libertyville, IL 60048 (US)
(72) Inventor: BOTTEN, Ronald, S., Libertyville, IL 60048 (US)
(74) Representative: FRKelly
(86) International application number: PCT/US2019/041126
(87) International publication number: WO 2020/014305

(56) References cited:
- EP-A2- 1 514 528
- US-B2- 7 981 099

## Description

### BACKGROUND

The following description relates generally to an ostomy appliance, and in particular, an ostomy pouch filter.

An ostomy pouch typically includes opposing sidewalls defining an internal collection area. One of the sidewalls is provided with an opening to receive a stoma, and means to secure the pouch to the user, such as an adhesive barrier, so that bodily waste discharged through the stoma is received within the collection area without leakage from the stoma/barrier/pouch environment.

An ostomy pouch may also include an ostomy pouch filter. A known ostomy pouch filter includes a single layer of deodorizing media, such as a carbon material. The ostomy pouch filter extends across an opening in the sidewall of the ostomy pouch to filter gas or odor exiting the ostomy pouch through the opening. However, with a single layer deodorizing media, an axial length of the gas flow path through the media is limited by a thickness of the media. Thus, to provide a desired level of odor filtering, a thickness of the media may need to be increased to a size that is bulky, heavy, or interferes with normal use of the ostomy pouch.

Another ostomy pouch filter is described in US Pat. No. 7,326,190 ("US '190") to Botten, which is commonly owned and assigned with the present application. In US '190, a porous filter pad has one or more passages into which a gas flows. The filter pad is disposed between axially and laterally spaced apertures. The relative positioning of the apertures and the filter pad creates a radial gas flow path through the filter pad, which may increase a length of the gas flow path through the filter pad and improve gas or odor filtering characteristics. However, such a radial flow filter may have a relatively large area or outline in the plan view.

Still another ostomy pouch filter is described in US Pat. No. 7,981,099 ("US '099") to Butler, which is commonly owned and assigned with the present application. In US '099, a filter assembly includes an envelope having first and second walls sealed at their edges to form a filter chamber, a multilayer filter pad having first and second layers of deodorizing filter media and an imperforate gas and odor barrier layer sandwiched between the filter layers. Further, the outer faces of the filter layers are covered by gas impermeable films or coatings sealed thereto. In one embodiment, the gas impermeable films or coatings may be omitted, and the walls of the envelope may perform the same functions instead. A gas-transmissible but liquid impermeable microporous membrane covers a central portion of an outer surface of the filter pad extending over an opening of the film.

However, the filter assembly of US '099 may be relatively complex and time consuming to manufacture. In addition, flow characteristics for a gas through the filter pad cannot be adjusted after manufacture of the filter pad because of the openings formed in the respective films extend over a fixed area. That is, a gas may only enter and exit the filter pad through a fixed area, after manufacture of the pad.

Accordingly, it is desirable to provide an ostomy pouch filter configured to provide desirable levels of gas and odor filtering through a radial flow path, that is relatively easy to manufacture and which allows for flow characteristics to be adjusted after manufacture of a filter body.

### SUMMARY

The invention is defined by claim 1 and relates to an ostomy pouch filter including a first layer of deodorizing media, a second layer of deodorizing media, a vapor barrier layer disposed between the first layer and the second layer, a first seal on a side of the first layer opposite to the vapor barrier layer, and a carrier film connected to the first layer with the first seal. The carrier film includes a gas flow path to allow for gas flow into and out of the first layer through a section of the carrier film. According to one embodiment, the ostomy pouch filter may further include a cover film.

According to another embodiment, an ostomy pouch includes a sidewall, and an ostomy pouch filter sealed to the sidewall. The ostomy pouch filter includes a first layer of deodorizing media, a second layer of deodorizing media, a vapor barrier layer disposed between the first layer and the second layer, a first seal on a side of the first layer opposite to the vapor barrier layer, and a carrier film connected to the first layer with the first seal. The carrier film includes a gas flow path to allow for gas flow into and out of the first layer through a section the carrier film. In one embodiment, the ostomy pouch filter may further include a cover film.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a cross-sectional view of an ostomy pouch filter according to an embodiment;
FIG. 2 is a cross-sectional view of a portion of an ostomy pouch having the ostomy pouch filter of FIG. 1, according to an embodiment;
FIG. 3 is a cross-sectional view of an ostomy pouch filter according to another embodiment; and
FIG. 4 is a cross-sectional view of a portion of an ostomy pouch having the ostomy pouch filter of FIG. 3, according to an embodiment.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

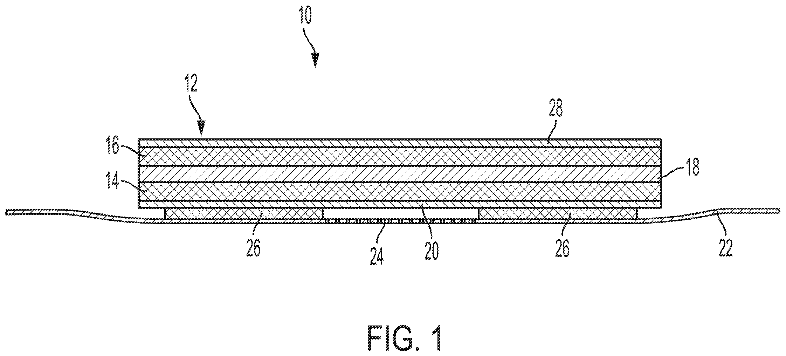

FIG. 1 is a cross-sectional view of an ostomy pouch filter 10 according to an embodiment. The ostomy pouch filter 10 described below and with reference to FIG. 1 may be referred to as a filter strip. The ostomy pouch filter 10 includes a filter body 12 comprising, generally, a first layer 14 of deodorizing media, a second layer 16 of deodorizing media and a vapor barrier layer 18 disposed between the first layer 14 and the second layer 16. In one embodiment, the first layer 14 and the second layer 16 may be similarly sized and shaped and made from the same material. For example, in one embodiment, the deodorizing media of the first layer 14 and the second layer 16 may be a carbon media that is gas permeable and configured to deodorize a gas flowing therethrough.

The vapor barrier layer 18 may be a film that is substantially gas impermeable. For example, in one embodiment, the vapor barrier layer 18 is a single or multilayer film. In one embodiment, the vapor barrier layer 18 may be formed from material(s), secured to adjacent layers of deodorizing media, include multiple layers and/or be formed in a manner similar to, or the same as, the imperforate gas and odor barrier layer described in the above-referenced US '099. For example, the vapor barrier layer 18 may be sandwiched between the first and second layers 14, 16 and sealed to inner faces of the same, thereby blocking axial flow of gases from one layer of deodorizing media to another. In one embodiment, the vapor barrier layer 18 may be composed of, for example, polyethylene or other polyolefin, or any other thin, flexible, preferably heat-sealable polymeric film known to have gas and odor barrier properties.

According to the invention, the filter body 12 further includes a first breathable membrane 20 disposed across the first layer 14 on a side of the first layer 14 opposite to the vapor barrier layer 18. In one embodiment, the first breathable membrane 20 is made from a heat sealable material, such as a non-woven material.

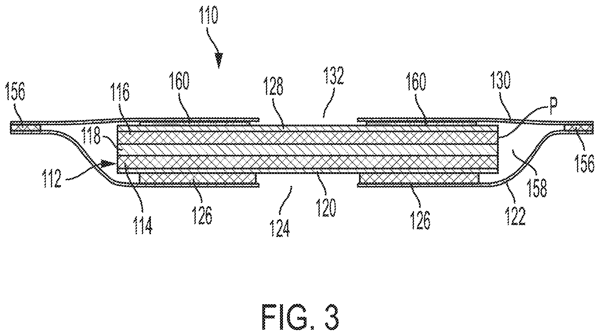

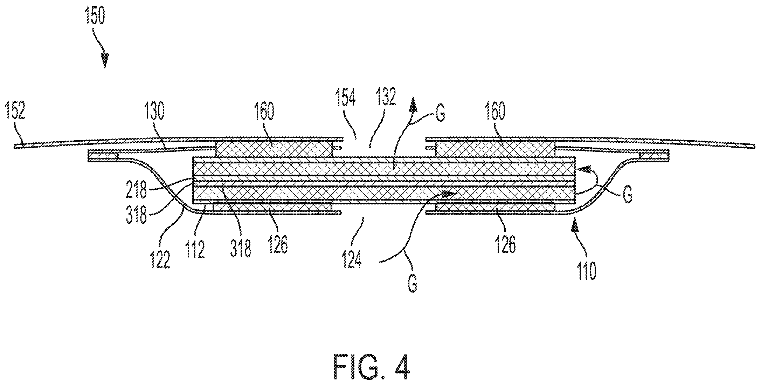

The ostomy pouch filter 10 may further include a carrier film 22. In one embodiment, the carrier film 22 is a film that is capable of being fed into an ostomy pouch machine and is connected to the first layer 14, for example, by sealing. In one embodiment, the carrier film 22 has a perforated section 24 to provide a gas flow path through the otherwise gas impermeable carrier film 22. For example, the carrier film 22 may be perforated by laser perforation at the perforated section 24. Alternatively, or in combination, the carrier film 22 may have one or more carrier openings 124 (see FIGS. 3 and 4) formed, for example, by punching or other known, suitable techniques, to provide the gas path. A remainder of the carrier film 22 may be gas impermeable. In one embodiment, the carrier film 22 is composed of a flexible plastic film or other known, suitable, preferably heat-sealable materials.

In one embodiment, the carrier film 22 may be sealed to the first layer 14 with a first seal 26 disposed between the carrier film 22 and the first breathable membrane 20. In one embodiment, a size of the first seal 26 affects a flow rate and a minimum distance traveled of a gas through first layer 14. The carrier film 22 may also protect the first layer 14 of deodorizing media.

The filter body 12 may further include a second breathable membrane 28 disposed on the second layer 16 on a side of the second layer 16 opposite to the vapor barrier layer 18. In one embodiment, the second breathable membrane 28 is made from a heat sealable material, such as a non-woven material.

Thus, in one embodiment, the filter body 12 may include the first breathable membrane 20, the first layer 14, the vapor barrier layer 18, the second layer 16 and the second breathable membrane 28. The first seal 26 may then be selected having a size, or area in the plan view of the filter body 12, which corresponds to desired flow characteristics of the gas through the filter body 12. For example, a first seal 26 having a relatively large area results in a smaller area through which gas may enter or exit the filter body 12. Such a smaller area may, for example, increase a minimum length of the gas flow path through the filter body 12. Conversely, a first seal 26 selected having a relatively small area results in a larger area through which gas may enter or exit the filter body 12. Such a larger area may, for example, decrease a minimum length of the gas flow path through the filter body 12. The carrier film 22 may then be secured to the filter body 12 with the first seal 26 as described above. In one embodiment, the area of the first seal 26 through which gas may enter or exit the filter body 12 is substantially aligned with the perforated section 24 or carrier opening 124 of the carrier film 22. In one embodiment, the first seal 26 may be substantially ring-shaped.

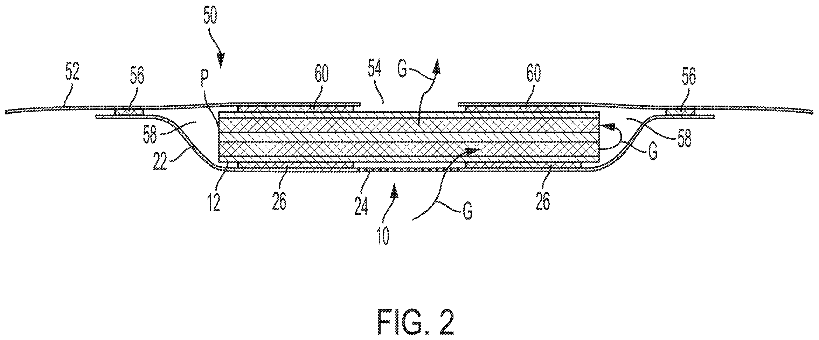

FIG. 2 is a cross-sectional view of a portion of an ostomy pouch 50 having the ostomy pouch filter 10 of FIG. 1, according to an embodiment. The ostomy pouch 50 includes a sidewall 52. Although not shown in FIG. 2, it will be appreciated by those skilled the art that the sidewall 52 defines an internal collection area (not shown) configured to receive bodily waste discharged from a stoma. The discharged bodily waste may be received through an inlet opening (not shown) formed in a sidewall on a body-facing side (not shown) of the ostomy pouch 50. The sidewall 52 includes a flow opening 54 adjacent to the ostomy pouch filter 10. In one embodiment, the ostomy pouch 50 and sidewall 52 may be formed the same, or substantially the same as the ostomy pouch and front and rear walls described in the above-referenced US '099, incorporated herein by reference. For example, in one embodiment, the sidewall 52 may be a front sidewall, and the ostomy pouch 50 may further include a rear sidewall (not shown), wherein the front and rear sidewalls are joined along their edges by heat sealing or by any other suitable means.

The ostomy pouch filter 10 may be attached to either an interior or exterior of the ostomy pouch 50. In one embodiment, the ostomy pouch filter 10 may be attached to the sidewall 52 of the ostomy pouch 50 at a peripheral location relative to the filter body 12 with a peripheral seal 56 between the carrier film 22 and the sidewall 52. Accordingly, a peripheral volume 58 may be formed that is bounded, at least in part, by an outer periphery P of the filter body 12, the carrier film 22 and the sidewall 52.

In addition, the sidewall 52 may be connected to the second layer 16 at a side of the second layer 16 opposite to the vapor barrier layer 18. In one embodiment, the sidewall 52 is connected to the second layer 16, for example, by a second seal 60 disposed between the second breathable membrane 28 and the sidewall 52. A size, or area, of the second seal 60 affects a flow path of the gas through the filter body 12 in a manner similar to that of the first seal 26 described above. In one embodiment, the second seal 60 may be substantially ring-shaped.

In the embodiments above, the first and second breathable membranes 20, 28, and the first and second layers 14, 16 of deodorizing media are gas permeable. The carrier film 22, the vapor barrier layer 18 and the first and second seals 26, 60 are gas impermeable. The carrier film 22 and first and second seals 26, 60 each include a section, however, through which gas may flow, such as an opening or perforated section. Accordingly, a tortuous gas flow path is provided by the ostomy pouch filter 10. That is, the gas may generally flow in an axial direction into and out of the ostomy pouch filter 10, and in a radial direction generally parallel to the vapor barrier layer 18 within the filter body 12. For example, as shown in FIG. 2, a gas G may flow through a section 24 (i.e., the gas path) of the carrier film 22 between the first seal 26, through the first breathable membrane 20 and into the first layer 14. In the first layer 14, the gas G is directed to flow substantially parallel to the vapor barrier layer 18 and exit the first layer 14 from an outer periphery P to flow into the peripheral volume 58. The gas G may then flow around the vapor barrier layer 18 into the second layer 16, through the second breathable membrane 28, and between the second seal 60 to be discharged through the flow opening 54 of the sidewall 52.

Accordingly, in the embodiments above, the tortuous gas flow path has an increased length compared to a flow path length in a similarly sized prior art filter having only a single layer of filter media. In addition, a length of the gas flow path may be adjusted depending on the selected size of the first and/or second seals 26, 60 after manufacture filter body 12.

FIG. 3 shows an example of an ostomy pouch filter 110 according to another embodiment. The ostomy pouch filter 110 of FIG. 3 may be referred to as a filter packet. The ostomy pouch filter 110 includes a filter body 112, which, in one embodiment, is substantially the same as the filter body 12 described above, unless described otherwise below. Accordingly, further description of the first layer 114 of deodorizing media, the second layer 116 of deodorizing media, and the vapor barrier layer 118 may be omitted below.

In one embodiment, the filter body 112 may further include a first breathable membrane 120 and/or a second breathable membrane 128 which are the same, or substantially the same as the first and second breathable membranes 20, 28 described in the embodiments above.

In one embodiment, the ostomy pouch filter 110 further includes the carrier film 122 and a first seal 126. These features substantially correspond to and are formed the same as the carrier film 22 and the first seal 26, respectively, as described above, unless described otherwise below. Thus, further description of the carrier film 122 and the first seal 126 may be omitted.

Referring to FIG. 3, the carrier film 122 may be formed with the one or more carrier openings 124 to allow for passage of the gas therethrough, either into or out of the filter body 112. Alternatively, or in addition, the carrier film 122 may be perforated, for example by laser perforation. As described above, the carrier film 122 may be sealed to the first layer 114 with a first seal 126 disposed between the carrier film 122 and first breathable membrane 120.

The ostomy pouch filter 110, according to an embodiment, further includes a cover film 130 extending over the second layer 116. The cover film 130 is gas impermeable and may include, for example, one or more cover openings 132 through which the gas may flow. Alternatively, or in addition, the cover film 130 may be perforated at a section, for example, by laser perforation to allow for gas flow through the section of the cover film 130. In one embodiment, the cover film 130 is composed of a flexible plastic film or other known, suitable, preferably heat-sealable materials. The cover film 130 may optionally be sealed to the filter body 112, for example at the second layer 116, with a second seal 160 positioned between the cover film 130 and the second layer 116. In one embodiment, the second seal 160 may be disposed between the cover film 130 and the second breathable membrane 128

The cover film 130 is connected to the carrier film 122 at an outer, peripheral region relative to the filter body 112, for example, with a peripheral seal 156. Accordingly, a peripheral volume 158 is formed around a periphery P of the filter body 122, and is bounded, at least in part, by the outer periphery P of the filter body 112, the carrier film 122 and the cover film 130.

FIG. 4 shows an example of an ostomy pouch 150 having the ostomy pouch filter 110 attached to a sidewall 152, according to an embodiment. The ostomy pouch 150 and the sidewall 152 are substantially same as those described above with reference to FIG. 2, unless described otherwise below. Referring to FIG. 4, as described above, the cover film 130 may be sealed to the second layer 116 with the second seal 160 disposed between the cover film 130 and the second breathable membrane 128. In one embodiment, the second seal 160 may also seal the cover film 130 to the sidewall 152. Thus, in one embodiment, the second seal 160 may be positioned on one side of the cover film 130 facing the second layer 116 and another side of the cover film 130 facing the sidewall 152. In one embodiment, the second seal 160 may be substantially ring-shaped. The ostomy pouch filter 110 may be connected to either an interior or exterior side of the sidewall 152. The sidewall 152 may include a flow opening 154 to allow the gas G to flow into or out of the ostomy pouch filter 110.

Accordingly, the ostomy pouch filter 110 described with reference to FIGS. 3 and 4 is configured to provide the tortuous gas flow path described with reference to the ostomy pouch filter 10 as well.

In the embodiments above, the filter body 12/112 may be formed, for example, by laminating the first and second layers 14/114, 16/116 and the vapor barrier layer 18/118. In one embodiment, the vapor barrier layer 18/118 includes a first vapor barrier layer 218 and a second vapor barrier layer 318. For example, during manufacture of the ostomy pouch filter 10/110, a first laminate may be formed by the first layer 14/114 of deodorizing media and the first vapor barrier layer 218. A second laminate may be formed by the second layer 16/116 of deodorizing media and the second vapor barrier layer 318. The first and second laminates are then positioned such that the first and second vapor barrier layers 218, 318 face one another, are brought into contact with one another and are joined together, for example, by heat sealing or other suitable method, including the use of known fasteners, such as an adhesive. Accordingly, the ostomy pouch filter 10/110 may be formed as described above, such that the vapor barrier layer 18/118 comprising first and second vapor barrier layers 218, 318 is disposed between the first layer 14/114 of deodorizing media and the second layer 16/116 of deodorizing media.

In the embodiments above, deodorization and gas flow performance or characteristics may be established by, for example, the choice of carbon media for the first and second layers 14/114, 16/116, the carbon media plan view shape and size, the carbon media thickness, inner and outer seal diameters of the first seal 26/126 between the carrier film 22/122 and the first layer 14/114 and inner and outer seal diameters of the second seal 60/160 between the cover film 130 or the sidewall 52 and the second layer 16/116.

In addition, in one embodiment, the cover film 130 may be more compliant than the carrier film 122, thereby allowing the cover film 130 to drape over the filter body 112 to create a flatter ostomy pouch filter 110. This configuration may improve sealability between the ostomy pouch filter 110 and the ostomy pouch 50. The cover film 130 may be more compliant than the carrier film 122, for example, by being formed of one or more different materials than the carrier film 122, having a different number of film layers than the carrier film 122, having a different dimensions (e.g., in a thickness direction) than the carrier film 122, and/or having one or more preformed locations allowing for flexibility (e.g., locations having reduced thickness). However, the present disclosure is not limited to such an example. In other embodiments, the cover film 130 and the carrier film 122 may be made more or less compliant depending on need or a particular application and may be equally compliant, or the carrier film 122 may be more compliant than the cover film 130.

Further, by way of using two carbon media layers, i.e., the first and second layers 14/114, 16/116, thinner pieces of carbon media may be used for each layer to achieve the desired level of odor filtering, compared to prior art ostomy pouch filters using a single piece of carbon media. The thinner pieces of carbon media may, collectively, be less expensive than a single, thicker piece of carbon media.

In one embodiment, the first and second breathable membranes 20/120, 28/128, the first and second layers 14/114, 16/116, and the vapor barrier layer 18/118 may have substantially the same footprint in the plan view, such that they are coextensive with one another and are flush, or substantially flush, with one another at the outer periphery P of the filter body 12/112. The first and second seals 26/126, 60/160 may have a footprint (area) less than the footprint (area) of the filter body 12/112 in the plan view.

In the embodiments above, where various films, layers or other components are described as being "sealed" to one another, or connected or secured to one another by a "seal," it will be appreciated that the seal may be formed as a direct seal, such as a heat seal or weld between the films, layers or other components, or may include an intermediate sealing material, such as an adhesive or other suitable material to effect the seal between the films, layers or other components.

In the present disclosure, the words "a" or "an" are to be taken to include both the singular and the plural. Conversely, any reference to plural items shall, where appropriate, include the singular. In additions, various features described with respect to any of the embodiments above may be used together, implemented in, or replace features in any of the other embodiments described above.

From the foregoing it will be observed that numerous modifications and variations can be effectuated without departing from the scope of the novel concepts of the present invention. It is to be understood that no limitation with respect to the specific embodiments illustrated is intended or should be inferred. The disclosure is intended to cover by the appended claims all such modifications as fall within the scope of the claims.

## Claims

1. An ostomy pouch filter (10, 110) comprising:
a first layer of deodorizing media (14, 114);
a second layer of deodorizing media (16, 116);
a vapor barrier layer (18, 118) disposed between the first layer and the second layer;
a first seal (26, 126) on the first layer on a side of the first layer opposite to the vapor barrier layer; and
a carrier film (22, 122) connected to the first layer with the first seal, wherein the carrier film includes a gas flow path (24, 124); and
**characterised in that** the ostomy pouch filter comprises a first breathable membrane (20, 120) on the side of the first layer opposite to the vapor barrier layer and positioned between the first layer and the first seal,
wherein an area of the first seal in a plan view is less than an area of the first layer in the plan view.

2. The ostomy pouch filter (10, 110) of claim 1, wherein the vapor barrier layer comprises two vapor barrier layers (18, 188, 318).

3. The ostomy pouch filter (10, 110) of claim 1 or 2, wherein the deodorizing media (14, 114, 16, 116) is carbon media.

4. The ostomy pouch filter (10, 110) of any of claims 1-3, wherein the carrier film is perforated.

5. The ostomy pouch filter (10, 110) of any of claims 1-4, further comprising a cover film (130) connected to the second layer.

6. The ostomy pouch filter (10, 110) of claim 5, wherein the cover film is connected to the second layer with a second seal (60, 160).

7. The ostomy pouch filter (10, 110) of claim 5 or 6, wherein the cover film is more pliable than the carrier film.

8. The ostomy pouch filter (10, 110) of any of claims 5-7, wherein the cover film is perforated to provide a gas flow path.

9. The ostomy pouch filter (10, 110) of claim 1, further comprising a second breathable membrane (28, 128) on a side of the second layer opposite to the vapor barrier layer.

10. The ostomy pouch filter (10, 110) of any of claims 5-9, wherein the cover film is sealed to the carrier film.

11. An ostomy pouch (50, 150) comprising:
a sidewall (52, 152); and
the ostomy pouch filter (10, 110) of any of claims 1 to 4.

12. The ostomy pouch (50, 150) of claim 11, wherein the carrier film is sealed to the sidewall.

13. The ostomy pouch (50, 150) of claim 11 or 12, further comprising a cover film connected to the second layer with a second seal.

14. The ostomy pouch (50, 150) of claim 13, wherein the cover film is sealed to the sidewall with the second seal.

15. The ostomy pouch (50, 150) of claim 13 or 14, wherein the cover film is sealed to the carrier film.

## Patentansprüche

1. Stomabeutelfilter (10, 110), umfassend:
eine erste Schicht aus desodorierenden Medien (14, 114);
eine zweite Schicht aus desodorierenden Medien (16, 116);
eine Dampfsperrschicht (18, 118), die zwischen der ersten Schicht und der zweiten Schicht angeordnet ist;
eine erste Dichtung (26, 126) auf der ersten Schicht auf einer Seite der ersten Schicht gegenüber der Dampfsperrschicht; und
eine Trägerfolie (22, 122), die mittels der ersten Dichtung mit der ersten Schicht verbunden ist, wobei die Trägerfolie einen Gasströmungsweg (24, 124) beinhaltet; und
**dadurch gekennzeichnet, dass** der Stomabeutelfilter eine erste atmungsaktive Membran (20, 120) auf der Seite der ersten Schicht gegenüber der Dampfsperrschicht aufweist, die zwischen der ersten Schicht und der ersten Dichtung positioniert ist,
wobei eine Fläche der ersten Dichtung in einer Draufsicht kleiner ist als eine Fläche der ersten Schicht in der Draufsicht.

2. Stomabeutelfilter (10, 110) nach Anspruch 1, wobei die Dampfsperrschicht zwei Dampfsperrschichten (18, 188, 318) umfasst.

3. Stomabeutelfilter (10, 110) nach Anspruch 1 oder 2, wobei es sich bei den desodorierenden Medien (14, 114, 16, 116) um Kohlenstoffmedien handelt.

4. Stomabeutelfilter (10, 110) nach einem der Ansprüche 1-3, wobei die Trägerfolie perforiert ist.

5. Stomabeutelfilter (10, 110) nach einem der Ansprüche 1-4, ferner umfassend eine mit der zweiten Schicht verbundene Abdeckfolie (130).

6. Stomabeutelfilter (10, 110) nach Anspruch 5, wobei die Abdeckfolie mittels einer zweiten Dichtung (60, 160) mit der zweiten Schicht verbunden ist.

7. Stomabeutelfilter (10, 110) nach Anspruch 5 oder 6, wobei die Abdeckfolie biegsamer ist als die Trägerfolie.

8. Stomabeutelfilter (10, 110) nach einem der Ansprüche 5-7, wobei die Abdeckfolie perforiert ist, um einen Gasströmungsweg bereitzustellen.

9. Stomabeutelfilter (10, 110) nach Anspruch 1, ferner umfassend eine zweite atmungsaktive Membran (28, 128) auf einer Seite der zweiten Schicht gegenüber der Dampfsperrschicht.

10. Stomabeutelfilter (10, 110) nach einem der Ansprüche 5-9, wobei die Abdeckfolie zu der Trägerfolie abgedichtet ist.

11. Stomabeutel (50, 150), umfassend:
eine Seitenwand (52, 152); und
den Stomabeutelfilter (10, 110) nach einem der Ansprüche 1 bis 4.

12. Stomabeutel (50, 150) nach Anspruch 11, wobei die Trägerfolie zu der Seitenwand abgedichtet ist.

13. Stomabeutel (50, 150) nach Anspruch 11 oder 12, ferner umfassend eine Abdeckfolie, die mittels einer zweiten Dichtung mit der zweiten Schicht verbunden ist.

14. Stomabeutel (50, 150) nach Anspruch 13, wobei die Abdeckfolie mittels der zweiten Dichtung zu der Seitenwand abgedichtet ist.

15. Stomabeutel (50, 150) nach Anspruch 13 oder 14, wobei die Abdeckfolie zu der Trägerfolie abgedichtet ist.

## Revendications

1. Filtre à poche de stomie (10, 110) comprenant :
une première couche de milieu désodorisant (14, 114) ;
une seconde couche de milieu désodorisant (16, 116) ;
une couche pare-vapeur (18, 118) disposée entre la première couche et la seconde couche ;
un premier joint (26, 126) sur la première couche sur un côté de la première couche opposé à la couche pare-vapeur ; et
un film porteur (22, 122) connecté à la première couche avec le premier joint, dans lequel le film porteur comprend un trajet d'écoulement de gaz (24, 124) ; et
**caractérisé en ce que** le filtre à poche de stomie comprend une première membrane respirante (20, 120) sur le côté de la première couche opposé à la couche pare-vapeur et positionné entre la première couche et le premier joint,
dans lequel une zone du premier joint dans une vue en plan est inférieure à une zone de la première couche dans la vue en plan.

2. Filtre à poche de stomie (10, 110) selon la revendication 1, dans lequel la couche pare-vapeur comprend deux couches pare-vapeur (18, 188, 318).

3. Filtre à poche de stomie (10, 110) selon la revendication 1 ou 2, dans lequel le milieu désodorisant (14, 114, 16, 116) est un milieu au carbone.

4. Filtre à poche de stomie (10, 110) selon l'une quelconque des revendications 1 à 3, dans lequel le film porteur est perforé.

5. Filtre à poche pour stomie (10, 110) selon l'une quelconque des revendications 1 à 4, comprenant en outre un film de couverture (130) relié à la seconde couche.

6. Filtre à poche de stomie (10, 110) selon la revendication 5, dans lequel le film de couverture est relié à la seconde couche avec un second joint (60, 160).

7. Filtre à poche de stomie (10, 110) selon la revendication 5 ou 6, dans lequel le film de couverture est plus pliable que le film de support.

8. Filtre à poche de stomie (10, 110) selon l'une quelconque des revendications 5 à 7, dans lequel le film de couverture est perforé pour fournir un chemin d'écoulement de gaz.

9. Filtre à poche pour stomie (10, 110) selon la revendication 1, comprenant en outre une seconde membrane respirante (28, 128) sur un côté de la seconde couche opposé à la couche pare-vapeur.

10. Filtre à poche de stomie (10, 110) selon l'une quelconque des revendications 5 à 9, dans lequel le film de couverture est scellé au film porteur.

11. Poche de stomie (50, 150) comprenant :
une paroi latérale (52, 152) ; et
le filtre à poche de stomie (10, 110) selon l'une quelconque des revendications 1 à 4.

12. Poche de stomie (50, 150) selon la revendication 11, dans laquelle le film support est scellé à la paroi latérale.

13. Poche de stomie (50, 150) selon la revendication 11 ou 12, comprenant en outre un film de couverture relié à la seconde couche avec un second joint.

14. Poche de stomie (50, 150) selon la revendication 13, dans laquelle le film de couverture est scellé à la paroi latérale avec le second joint.

15. Poche de stomie (50, 150) selon la revendication 13 ou 14, dans laquelle le film de couverture est scellé au film porteur.
